# EUROPEAN PATENT SPECIFICATION

(11) **EP 4 041 091 B1**
(45) Date of publication and mention of the grant of the patent: **21.08.2024**
(21) Application number: 20796701.9
(22) Date of filing: 07.10.2020
(51) Int. Cl.: A61B 10/00, G01N 1/20

(54) **SMALL VOLUME LIQUID SAMPLER**
KLEINVOLUMIGER FLÜSSIGPROBENNEHMER
ÉCHANTILLONNEUR DE LIQUIDE DE PETIT VOLUME

(30) Priority: 07.10.2019 US 201962911817 P
(43) Date of publication of application: 17.08.2022
(73) Proprietor: Novosanis NV, 2110 Wijnegem (BE)
(72) Inventor: BEYERS, Koen, Catharina, Lodewijk, 2990 Wuustwezel (BE); RÍOS CORTÉS, Alejandra, 3070 Kortenberg (BE); MEERS, Nette, 2500 Lier (BE); BAKS, Christof, Irma, Jozeph, 2160 Wommelgem (BE); VAN AVONDT, Quinten, 2500 Lier (BE); VAN MULDER, Timothi, Julio, Steven, 2970 Schilde (BE); VANKERCKHOVEN, Vanessa, Vicky, Jill, 2610 Wilrijk (BE)
(74) Representative: Winger
(86) International application number: PCT/EP2020/078023
(87) International publication number: WO 2021/069454

(56) References cited:
- DE-A1- 1 903 251
- DE-A1- 2 327 699
- GB-A- 1 574 864
- KR-B1- 101 876 699
- US-A1- 2007 074 761
- US-A1- 2011 284 220
- US-A1- 2015 223 784

## Description

### Field of the invention

The present invention relates to the field of fluid volume sampling devices, and in particular to devices and kits for sampling small initial volumes of a liquid flow.

### Background of the invention

With the development of highly sensitive nucleic acid amplification testing of pathogenic DNA, self-collected first-void urine has become a valuable non-invasive sample for diagnostic purposes, for instance for the detection of urogenital infections such as Chlamydia trachomatis, as well as other sexually transmitted infections. Results of this testing method, however, are only conclusive if the sampled urine fraction is not diluted or contaminated by the subsequent mid-stream urine. Moreover, even within the first-void volume of urine, there are variations of the microorganism load in urine samples which depend on the precise initial volume sampled, e.g. only a small fraction of the first-void urine. Therefore, a need for liquid sampling devices exists which allow the precise sampling of an initial volume of a liquid flow, e.g. urine, and which are hygienic and comfortable in their use.

WO2014/037152 (and family member US 2015/223784 A1) relates to a liquid sampling device for capturing a first portion of a liquid flow. The device comprises an inlet, an outlet, and a guide with a displaceable element which, in a first position, is capturing a first portion of the liquid flow, e.g. the first-void urine, into a reservoir, and which, in a second position, is blocking the access to the reservoir and is passing subsequent liquid to the outlet instead. The displaceable element moves in transverse direction to the liquid flow and has lifting means. Although being suitable for the sampling of first-void urine, it remains challenging for this device to restrict the quantity of sampled first-void urine to a small initial volume fraction thereof. The preambles of the independent claims are based on said document.

KR101876699 describes a urine sample collector comprising: a body housing including an inlet part to introduce urine, a first flow path part to provide an initial flow path for the introduced urine, and an outlet part to discharge the urine, wherein a flow path between the first flow path part and the outlet part is cut off; a sample storage part to provide a space for storing a predetermined amount of a urine sample from the urine; and a floating unit providing a second flow path part which provides a second flow path to connect the flow path between the first flow path part and the outlet part, wherein the first flow path part provides a first flow path to move the urine discharged from the first flow path part into the sample storage part at a first position and to move the same from the first position to a second position by buoyance generated by a volume increase of the urine sample collected in the sample storage part to be cut on the second position, so as to move the urine discharged from the first flow path part to the outlet part. Also for this device it is challenging to restrict the quantity of sampled first-void urine to a small initial volume fraction thereof.

GB1574864 describes a liquid collector for collecting mid stream samples.

US2011/0284220, DE2327699, DE1903251 and US2007/0074761 relate generally to sampling of liquids.

### Summary of the invention

Miniaturizing buoyancy-driven liquid sampling devices described in WO2014/037152 A1 to make them fit to smaller volumes as required for diagnostics is challenging because the buoyancy force necessary to elevate the displaceable element (e.g. a valve) is strongly reduced for smaller-sized lifting means, such as air pockets or floaters, which still fit into the sampling receptacles. Another problem faced by miniaturization is the limited wall thickness that is yet obtainable by injection molding techniques, e.g. limited to 0.5 mm. There are indeed chances that designs with limited use of material, thus very thin-walled designs, with a wall thickness below 0.5 mm, e.g. 0.4 mm thick or thinner, are facing filling issues in the mold when they are injection molded. Further, the redirection of the sampled liquid volume through tubular ducts of small diameter is slow and possibly dominated by capillary action, which can result in liquid rapidly accumulating in the chamber (e.g. guide portion) connected between the inlet and outlet conduits. As liquid is held back and rises in the chamber, the inflow of the initial fraction is hampered and retarded. Ultimately, the subsequent volume may dilute or mix or contaminate with the initial volume to be sampled if the latter is not drained fast enough.

It is therefore an object of embodiments of the present invention to provide a device for sampling an initial volume of a liquid flow, hygienic and comfortable in use, which is also adapted for reliably sampling small initial volumes, e.g. volumes smaller than 20 ml, e.g. about 5 ml or less.

The above objective is accomplished by a device and a kit of parts according embodiments of to the present invention.

In one aspect the invention relates to a device for sampling an initial volume of a liquid flow and the device comprises an inlet conduit for receiving the liquid flow, an outlet conduit for draining a subsequent volume of the liquid flow, and a valve casing arranged between the inlet conduit and the outlet conduit. The valve casing has a passageway formed therethrough to fluidly connect the inlet conduit to the outlet conduit, for instance is providing an inlet and an outlet through a projecting sidewall. A sample outlet for draining the initial volume of the liquid flow is extending through the valve casing and into said passageway. Further, a valve structure is arranged for axial movement in the valve casing, traverse to a direction of liquid flow through the passageway. The valve structure comprises a gate for obstructing the passageway and for preventing the initial volume of the liquid flow from being transferred to the outlet conduit when the valve structure is in, e.g. moved to, a sampling position. Besides, the valve structure comprises a stem which is connected to the gate and adapted for obstructing the sample outlet without obstructing the passageway when the valve structure is in, e.g. moved to, a diverting position. At least one elongated groove is formed in a surface of the gate that faces the inlet conduit. The at least one groove extends from the gate into the stem such that the initial volume of the liquid flow is directed through the sample outlet and towards a receptacle, which is connectable to the valve casing, when the valve structure is in, e.g. moved to, the sampling position. The at least one elongated groove forms a narrow channel in the gate and the stem, which is fit to receive the inflowing initial volume and to guide it in a downward movement along the gate and the stem, and through the sample outlet if the valve structure is moved to the sampling position. The at least one elongated groove thus enables a liquid passageway for the initial volume flow between the inlet conduit and the sample outlet when the valve structure is in the sampling position. Moreover, a buoyancy-driven lifting member is connected to the stem for moving the valve structure from the sampling position to the diverting position while the initial volume of the liquid flow is being sampled through the sample outlet.

According to some embodiments of the present invention, the gate includes a slanted wall for achieving obstruction of the passageway through the valve casing and for obtaining an additional lifting force at the same time.

In some embodiments of the present invention, a resilient stopper is protruding from the inner surface of the valve casing to prevent a vertical displacement of the valve structure beyond the diverting position. Various height positions of the stopper can be determined to match a predetermined initial volume that is sampled in standardized receptacles, e.g. test tubes.

In some embodiments of the present invention, the rim of the cap and/or the outer edge of a lower portion of the stem is fitted (e.g. complementary in size and shape) to lie flush with a corresponding surface of the valve casing and the bore of the sample outlet respectively. This provides an efficient sealing effect.

In some embodiments of the present invention, the cap and/or ribbed edges of the valve structure are in a telescopic relationship with the inner surface of the valve casing and the bore of the sample outlet respectively. This improves a sliding axial movement of the valve structure relative to the valve casing by a guiding assistance at reduced friction.

According to some embodiments of the present invention, the cap has an asymmetrical shape (e.g. defined by its rim) to prevent a rotation movement of the valve structure around its axis of translation. Hereto, the valve structure may have a mating asymmetrical shape.

It is an advantage of embodiments of the present invention that the device can cope with a wide range of volumetric flow rates, including volumetric flow rates typical for urination, e.g. ranging from 1 ml/sec to 55 ml/sec.

It is an advantage of embodiments of the present invention that components of the device may be composed of biodegradable polymers or other suitable biomaterials, which can be readily disposed of after use.

It is an advantage of embodiments of the present invention that inlet conduits and/or funnels can be adapted for use by a specific gender. This increases user comfort and also reduces the risk of spillage.

It is an advantage of embodiments of the present invention that the components of the sampling device can be manufactured separately and assembled later. Hence, the components of the sampling device can be sent to the user as a kit of parts, involving only little shipment costs and the user can take samples at home.

In a further aspect the invention relates to a kit of parts, for assembling a device according to embodiments of the first aspect, which comprises a bonnet with at least three openings, an inlet conduit that is connectable to a first opening of the bonnet, an outlet conduit that is connectable to a second opening of the bonnet, and a valve casing that is removably insertable into the bonnet via a third opening therein. A base of the valve casing is connectable to the third opening, and the valve casing has an inlet and an outlet formed therethrough, defining a liquid passageway though the valve casing. A sample outlet extends through the base of the valve casing and into the passageway. Additionally, the valve casing has an opening provided at a top side, opposite to the base through which a valve structure is insertable. A valve structure, also included in the kit, is removably insertable into the valve casing. The valve structure is arranged for axial movement in the valve casing, traverse to a direction of liquid flow through the passageway. A gate of the valve structure is configured for obstructing the passageway when the valve structure is in, e.g. moved to, a sampling position, a stem of the valve structure is connected to the gate and adapted for obstructing the sample outlet without obstructing the passageway when the valve structure is in, e.g. moved to, a diverting position. At least one elongated groove is formed in a surface of the gate and extends into the stem. The at least one groove is adapted for, when the kit of parts is assembled and in use, receiving an initial volume of a liquid flow and for guiding said initial volume along the gate, the stem, and through the sample outlet towards a receptacle, connectable to the valve casing, when the valve structure is in a sampling position. Moreover, the kit includes a buoyancy-driven lifting member that is connectable to the stem.

Alternatively, a kit of parts comprises a valve casing with an inlet and an outlet formed therethrough, defining a liquid passageway through the valve casing. The kit further comprises a sample outlet which extends through a base of the valve casing and into the passageway, and an inlet conduit and outlet conduit which are connectable or connected to the inlet and the outlet of the valve casing respectively. An opening is provided at a top side of the valve casing, opposite to the base. Besides, a valve structure is included in the kit which is removably insertable into the valve casing. The valve structure is arranged for axial movement in the valve casing, traverse to a direction of liquid flow through the passageway. A gate of the valve structure is adapted for obstructing the passageway when the valve structure is in, e.g. moved to, a sampling position, and a stem of the valve structure is connected to the gate and adapted for obstructing the sample outlet without obstructing the passageway when the valve structure is in, e.g. moved to, a diverting position. At least one elongated groove is formed in a surface of the gate and extends from the gate into the stem. The at least one groove is adapted for, when the kit of parts is assembled and in use, receiving an initial volume of a liquid flow and for guiding said initial volume along the gate, the stem, and through the sample outlet towards a receptacle, connectable to the valve casing, when the valve structure is in a sampling position. Furthermore, the kit comprises a buoyancy-driven lifting member that is connectable to the stem.

Particular and preferred aspects of the invention are set out in the accompanying independent and dependent claims. Features from the dependent claims may be combined with features of the independent claims and with features of other dependent claims as appropriate and not merely as explicitly set out in the claims.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### Brief description of the drawings

The invention will now be described further, by way of example, with reference to the accompanying drawings, in which:
FIG. 1 is a cross-sectional view of a device for sampling an initial volume of a liquid flow according to an embodiment of the invention.
FIG. 2 and FIG. 3 are side and front elevation views of a valve structure as used in an embodiment of the invention.
FIG. 4 and FIG. 5 are perspective views for the valve structure shown in FIG. 2 and FIG. 3.
FIG. 6 is an exploded view of a kit of parts comprising a device for sampling an initial volume of a liquid flow according to an embodiment of the invention.
FIG. 7 is an exploded view of a kit of parts comprising a device for sampling an initial volume of a liquid flow according to a different embodiment of the invention.

In the drawings, the size of some of the elements may be exaggerated and not drawn on scale for illustrative purposes. The dimensions and the relative dimensions do not necessarily correspond to actual reductions to practice of the invention.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims.

Moreover, directional terminology such as top, bottom, front, back, leading, trailing, under, over and the like in the description and the claims is used for descriptive purposes with reference to the orientation of the drawings being described, and not necessarily for describing relative positions. Because components of embodiments of the present invention can be positioned in a number of different orientations, the directional terminology is used for purposes of illustration only, and is in no way intended to be limiting, unless otherwise indicated. It is, hence, to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. The scope of the invention is defined by the appended claims solely.

It should be noted that the use of particular terminology when describing certain features or aspects of the invention should not be taken to imply that the terminology is being re-defined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

When reference is made in embodiments of the invention to a first-void volume of urine, this generally designates the first 20 ml to 50 ml of the initial urine flush. Small volumes in this respect, refer to volumes of first-void volume which are smaller than 20 ml, e.g. less than 10 ml, e.g. less than 5 ml, such as for instance 4 ml.

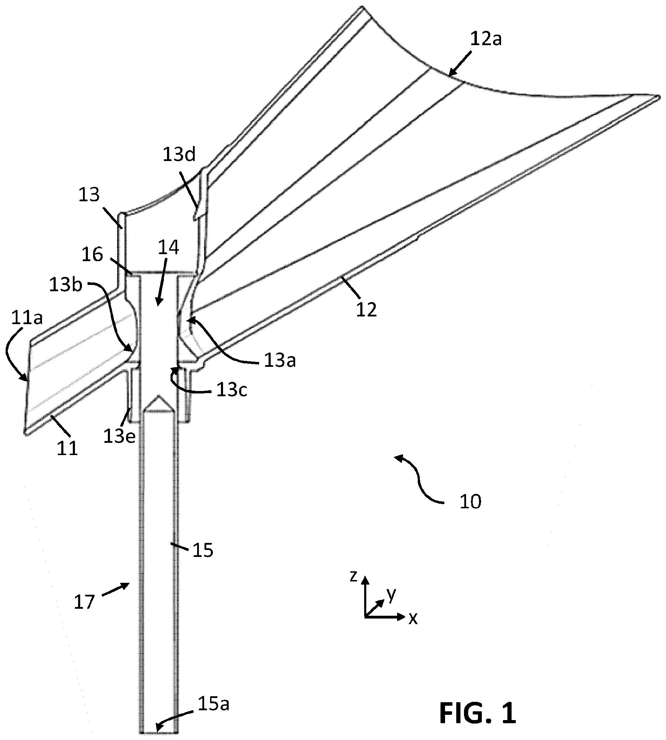

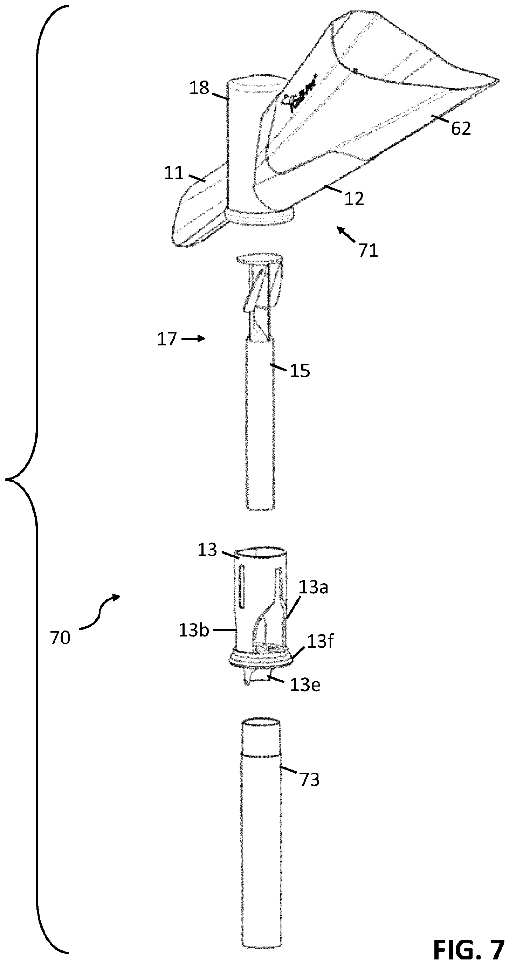

With reference to FIG. 1 to FIG. 5 of the drawings, a device for sampling an initial volume of a liquid flow (consisting of an initial volume and a subsequent volume) according to an embodiment of the invention is now described. FIG. 1 shows a cross-sectional view of the device 10, in which the section is taken in a mirror plane of the device perpendicular to the y-axis. The device 10 comprises an inlet conduit 12, an outlet conduit 11, a valve casing 13 and a valve structure 17. The valve structure 17 is inserted into the valve casing 13 and arranged to move axially inside the valve casing 13 between a sampling position and a diverting position. The inlet conduit 12 is adapted for receiving a liquid flow, e.g. urine, at a distant opening 12a and for guiding it towards the valve casing 13 and the outlet conduit 11 is adapted for draining the subsequent volume of the liquid flow, e.g. urine, away from the valve casing 13 and for expelling it from the device 10 at a distant opening 11a. Although the inlet conduit 12 and the opening 12a can be shaped in numerous ways, an inlet conduit 12 forming a funnel is preferred for the purpose of collecting the liquid efficiently and without spillage; particularly for the sampling of urine, the funnel-shaped inlet conduit 12 with a widened receiving lip as opening 12a offers the user a more hygienic and more comfortable use of the device 10. Moreover, the inlet conduit 12 and the outlet conduit 11 are preferably arranged at a slanting position with respect to a vertical z-axis along which the elongated valve structure 17 is typically aligned when the device 10 is operated, e.g. at angles ranging between 20 arc degrees and 70 arc degrees, e.g. about 45 arc degrees. This has the advantage that a liquid flow which enters the device 10 at the opening 12a at a low flow velocity is forced onward without delay, whereby a faster sampling of the initial volume thereof is achieved while contamination and sticking of the liquid to the inlet conduit 12 walls is prevented. Similarly, the removal of the subsequent volume of the received liquid flow from the valve casing 13 is accelerated by the slanted outlet conduit 11, thereby reducing the risk of contaminating the sampled initial volume by residuals of the subsequent volume remaining in the valve casing 13. The valve casing 13 has an inlet 13a and an outlet 13b formed therethrough, e.g. formed as openings in a projecting wall (in the z-direction) of the valve casing, a flat base supporting the projecting wall at a lower side, and an opening at an upper side, which opening is adapted for receiving the valve structure 17. A passageway for the liquid extends between the inlet 13a and the outlet 13b and through the hollow interior of the valve casing 13, wherein the inlet 13a and the outlet 13b are permanently aligned with proximate openings of the inlet conduit 12 and the outlet conduit 11respectively. The proximate openings of the inlet conduit 12 and the outlet conduit 11 may be of equal shape and size as the inlet 13a, respectively the outlet 13b of the valve casing 13, or may be shaped differently and/or differ in size. In this particular embodiment, this permanent alignment is achieved by providing the inlet conduit 12, the outlet conduit 11, and the valve casing 13 as a single, monolithically formed unit, in which the inlet conduit 12 as well as the outlet conduit 11 are directly connected to the projecting wall of the valve casing 13. However, this way of connecting the inlet and outlet conduits 12, 11 to the valve casing 13 is not limiting and other connection means may be provided instead. For instance, a snap-fit connection may be made between plastic pieces, or push-in fittings or compression fittings may be used to connect the valve casing 13 to soft or hard tubing used for the inlet and outlet conduits 12, 11. A different connection scheme between valve casing 13 and inlet and outlet conduits 12,11 is described further below with reference to FIG. 7 in relation to an alternative embodiment. A sample outlet 13c, e.g. a circular opening, is formed through the valve casing 13 at the flat base thereof and is leading into the passageway between the inlet 13a and outlet 13b.

The valve structure 17, as shown in FIG. 1, is in an inserted configuration, in which at least a gate 14 of the valve structure 17 is received inside the valve casing 13, e.g. in the hollow interior defined by the projecting walls, and an elongated stem 15 of the valve structure 17 is extending through the sample outlet 13c. Besides, the valve structure 17 is arranged for an axial movement in the valve casing 13 from a sampling position to a diverting position. For the embodiment described in reference to FIG. 1, the axial movement corresponds to a linear movement along the vertical direction (e.g. z-axis) and in a direction traverse to the direction of flow through the passageway between inlet 13a and outlet 13b, e.g. in a direction substantially perpendicular to the direction of flow through the passageway. Notwithstanding the preferred ninety degree angle between the axial movement of the valve structure 17 and the direction of flow through the passageway (e.g. defined by an inclination angle of the flat base of the valve casing about the z-axis), embodiments of the invention are not limited to ninety degree angle or angles close to the ninety degree angle. For instance, the flow direction for an inclined base may be slanted and the resulting angle between the flow direction through the passageway and the axial movement of the valve structure 17 may take values in the range (90 +/- 25) arc degrees. In this particular embodiment, a lifting member 15a is provided as an air-filled elongated cavity formed in the lower portion of the stem 15 and extending in the direction of axial movement of the valve structure 17, e.g. in the vertical z-direction. However, different embodiments of the invention may be provided with a different lifting member, for instance a block connected to an end portion of the stem or a block fastened to the stem and surrounding the same, which block comprises one or more air pockets, air-filled cavities, or comprises a porous material, e.g. a foam (e.g. an extruded polystyrene foam).

The sampling position, which is illustrated in FIG. 1, is adopted if the gate 14 is contacting the base of the valve casing 13 and a farther downward movement of the valve structure 17 with respect to the stationary valve casing 13 is prevented. More specifically, the valve structure is moved to the sampling position if a laterally outwards extending portion of a bottom edge of the gate 14 is physically contacting an upper side of the base in a region that is not overlapping the sample outlet 13c, e.g. the base portion surrounding the sample outlet 13c is acting as a seat for the gate 14. Moved to the sampling position, the gate 14 of the valve structure 17 is obstructing the liquid flow of the initial volume through the passageway between the inlet 13a and the outlet 13b.

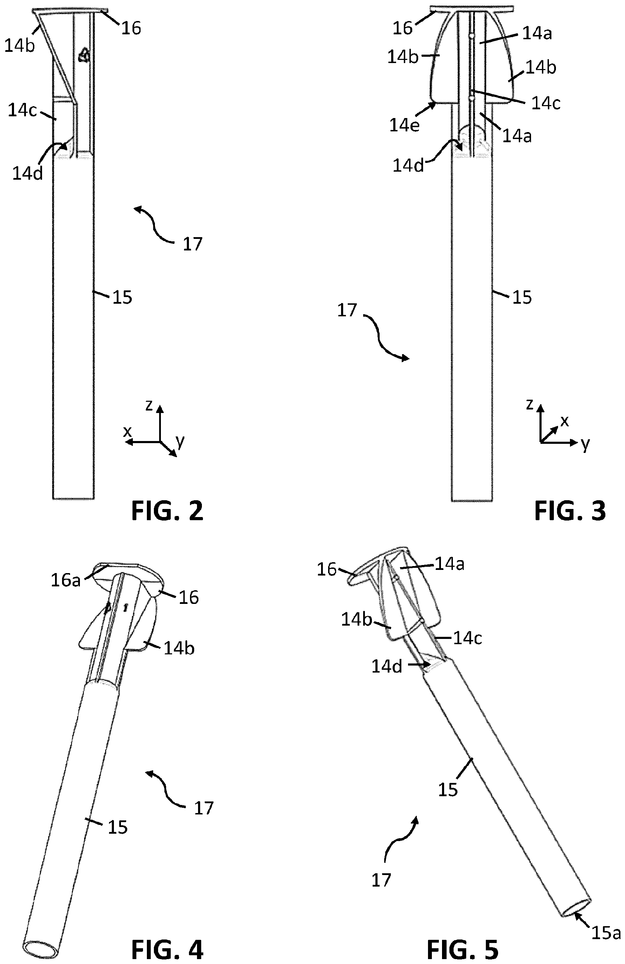

This obstruction mechanism of the gate 14 of the valve structure in the sampling position can be more readily understood by referring to FIG. 2 and FIG. 3, which are more detailed side elevation and front elevation views of the valve structure 17 respectively. In particular, the skilled person's attention is drawn to the presence of a slanted wall 14b of the gate 14 at a frontal side thereof. The wall 14b laterally extends, in the shape of two winged pieces, from a wedge-tapered central shaft, which is a continuation of the stem 15 in an upward direction. The slope angle of the wall 14b may be determined as the angle of intersection between a first plane that contains the base of the valve casing and a second plane, intersecting the first plane, which contains the diameter in y-directions across the sample outlet 13c in the base and the apex (in z-direction) of the arced curve defining the contour of the inlet 13a. A bottom edge 14e of the wall14b joins the central shaft in the upper, thinned portion of the stem 15, above a beveled (e.g. conical) transition segment 14d, and the top edge of the wall is delimited by a flat cap 16. The wall 14 b may have a spade-like contour and resemble a corresponding spade-like contour of a beaked inlet 13a of the valve casing 13. In particular, the projected contour of the wall 14b preferably covers the contour of the inlet 13a when projected onto a frontal plane. Furthermore, the contour of the wall 14b may be defined by the curve obtained by cutting a cylinder (e.g. for a cylindrical valve casing) with the second plane mentioned above. At least one groove 14a is formed in the surface of the wall 14b and extends towards and into the surface of the central shaft in the upper, thinned portion of the stem 15. The at least one groove 14a is more easily distinguished in the perspective view of the valve structure in FIG. 5. As shown in FIG. 2, FIG. 3 and FIG. 5, the at least one groove 14a may be considered as two separate grooves in the presence of a thin upwards raising, second wall 14c, which divides the groove space into two halves. This additional separating wall 14c has the advantage of increasing the structural integrity of the valve structure 17. The numerous details presented for the valve structure 17, help elucidating the obstruction mechanism for the gate 14 of the valve structure in the sampling position inside the valve casing 13. When the gate 14 is moved to the sampling position, the bottom edge 14e of the wall 14b are contacting the upper side of the valve casing base and the lateral winged edges of the wall 14b lie flush with the inner surface of the projecting wall of the valve casing 13, thereby providing the passageway through the valve casing with a sealing effect relative to the initial volume of the liquid flow. Moreover, the rim of the valve structure 17 is provided by the outwards directed edge of the cap 16. It is an advantage of embodiments of the invention that the cross-section of the cap 16 (in planar view, perpendicular to the z-axis) can be fitted to match, in shape and size, the inner boundary of the valve casing projecting wall such that the axial movement of the valve structure relative to the stationary valve casing is assisted and guided by the cap, e.g. the inner surface of the projecting wall of the valve casing 13 and the cap 16 are in a telescoping relationship. It is noted that the mentioned guiding aspect can be further improved by fitting the outer diameter of the stem in y-direction to the inner diameter of the sample outlet 13 in the same direction, which then also exhibit a telescoping relationship for their edges at least in the y-direction. As a result of the improved guiding of the valve structure 17 at two different heights (in z-direction), a tilting movement of the valve structure 17 can be prevented while still be able to move axially. In addition thereto, the so fitted cap 16 provides enhanced sealing of the passageway in respect of the initial volume of the received liquid flow, but also in respect to splashes or spilling of the subsequent volume of the received liquid flow, e.g. urine, when it is directed through the passageway to be expelled at the outlet conduit opening 11a. The skilled artisan knows, by design routine or trial and error, how to select appropriate clearances between the cap and the inner surface of the projecting wall, between the contours of the wall 14b and the inner surface of the projecting wall, as well as between the stem and the sample outlet, such that the guiding is performed without significant friction and yet amenable to sealing off the passageway.

As mentioned, the initial volume is prevented from entering the outlet conduit 11, hence well-preserved for sampling, if the valve structure 17 is moved to the sampling position and the passageway between the inlet 13a and the outlet 13b of the valve casing is blocked. However, there exists a fluid connection between the inlet 13a and the sample outlet 13c as long as the initial volume of the liquid flow is received and sampled. More specifically, the at least one groove 14a formed in the wall 14b of the gate 14 ensures that the received initial volume is redirected towards the sample outlet 13c. Because the at least one groove 14a (e.g. one single groove or two grooves separated by a wall 14c) extends downwards beyond the bottom edge 14a of the wall 14b and into the upper, thinned portion of the stem 15, the redirected flow of the initial volume indeed traverses the sample outlet 13c and continuous flowing downwards along the outer surface of the stem 15. This is facilitated by the smooth beveled transition segment 14d of the stem 15, which avoids that the redirected flow of the initial volume is projected back into the valve casing 13 if the transaction to the lower, wider portion of the stem 15 was too abrupt. In consequence, also a reaction force encumbering the swift lifting of the valve structure 17 to the diverting position to ensure a small sample volume is significantly reduced. Moreover, the slanted wall 14b also contributes to the redirection of the flow of the initial volume towards the base of the valve casing 13, where the redirected flow can still be drained through the sample outlet 13c owing to the thinned portion of the stem 15, e.g. the thinned cross-sectional area of the upper portion of the stem, between the transition segment 14d and the bottom edge 14e of the wall 14b, compared to the wider cross-sectional area of the lower portion of the stem 15 (as seen in planar view, perpendicular to the z-axis). A further advantage of the slanted wall 14b is given by the fact that the received initial volume of the liquid flow is exerting a (dynamic) pressure force on the wall 15b that is decomposed into a force component parallel to the flow direction and a force component parallel to the z-axis, e.g. the slanted wall 14b can be used to further enhance an upwards directed lifting force, in addition to the buoyancy force through the lifting member 15a, which is driving the valve structure 17 from the sampling position to the diverting position. This secondary lifting force by the slanted wall 14b is much appreciated in cases of small sampled initial volumes, e.g. about 4 ml of first-void urine, for which a volume of the lifting member that is submerged in the already sampled and collected liquid is typically small, hence achieving only small buoyancy forces, as volumes of collecting receptacles cannot be made arbitrarily small. Receptacles of too small volume will otherwise render capillary adhesion forces decisive and the initial volume is not removed quickly enough to prevent contamination thereof by the subsequent volume, e.g. contamination of an earlier fraction of the first-void urine by a later fraction of first-void or mid-stream urine.

Preferably, a tubular sampling connector 13e extends downwards from a lower side of the base of the valve casing 13, at least partially circumferentially enclosing the opening of the sample outlet 13c. A receptacle for collecting the initial volume of the sampled liquid flow, may be removably connectable to said sampling connector 13e, e.g. via a snap-fit or screw-fit connection. Gravitational acceleration forces assist in draining the initial volume through the sample outlet 13c, along the outer surface of the stem 15, and towards the receptacle that is connected to the sampling device 10 prior to use. It is advantageous to provide the sampling connector 13e with a recess over a portion of its circumference to obtain a sampling connector with a baffle-type end portion since this further assists and accelerates the draining process of the initial volume from the lower end of the groove 14a over the beveled transition segment 14d towards the outer surface of the lower portion of the stem 15, whereby the risk of sticking and congestion by accumulation of the drained liquid is reduced.

During use of the device 10, the liquid flow pertaining to the initial volume is sampled through the sample outlet 13c and generates a liquid column in a receptacle that has been previously connected to the device 10, e.g. to the sampling connector 13e. Preferably, the shape and depth of the receptacle is selected to cooperate with the lifting member 15a in that the formation of the liquid column in the receptacle is accompanied by a quickly progressing immersion of the lifting member 15a. The lifting member 15a may be provided as an air-filled cavity that is arranged in the hollow interior of the lower portion of the stem 15. This has the additional advantage that material weight and cost related to the formation of the elongated stem 15 is conveniently saved by provision of the cheap, low density fluid air. This also allows connectable receptacles of smaller diameter to be used to efficiently increase the immersed volume of the lifting member 15a, hence to increase the buoyancy force. Nevertheless, embodiments of the invention are not limited thereto and other floating structures and/or materials may be used instead of, or in addition to, air cavities in the lower portion of the stem 15, for instance a block of foams into which the lower end portion of the stem extends. As a consequence of the buoyancy force acting on the lifting member 15a as a floater, the valve structure 17 as a whole is lifted and forced upwards as the height of the sampled liquid column continues raising. The buoyancy-driven vertical displacement is stopped, and a further elevation of the valve structure 17 prevented, as soon as the diverting position is adopted.

In the diverting position, the upper side of the cap 16 is pushed up against a resilient stopper 13d, a projection protruding from the inner surface of the valve casing 13, e.g. a small flexible pin or stud extending inwards into the valve casing bore. The cap 16 is provided as a asymmetrically shaped disk, e.g. an originally circular disk of which a segment has been removed along a chord, which is most visible in the perspective view of FIG. 4. Hence, the rim of the cap 16 is lying flush with the inner side of the projecting walls of the valve casing 13 in the sampling position, in the diverting position, and in all the positions along the axial movement of the valve structure 17, whereby a sealing and guiding effect are obtained. Moreover, the asymmetrical shapes of the cap 16 and the bore of the valve casing 13 (e.g. the hollow interior defined by the projecting walls) are adapted to prevent a rotation movement around the vertical axis of displacement. Other shapes for the cap and the bore can be selected to yield the same effect, e.g. elliptical shape, polygonal shape, etc. The resilient stopper 13d is formed at a height (in z-direction) which corresponds to the buoyancy-driven elevation of the valve structure 17 when the initial volume has been drained through the sample outlet 13c. For high volume flow rates of the liquid to be sampled particularly, the sample outlet 13c aperture size and/or slanted wall 14b assist in obtaining a swift transition of the valve structure 17 from the sampling position to the diverting position, whereby the subsequent volume of the liquid flow is effectively prevented from passing through the sample outlet 13c and from contaminating the initial volume sample. It is further noticed that the thinned portion of the stem connected between the bottom edge 14e of the gate wall(s) 14b and the transition segment 14d, and the reduced lateral dimension in y-direction in particular (cf. FIG. 3), permits the subsequent volume of the liquid flow to be diverted through the passageway of the valve casing 13, between the inlet and outlet 13a, 13b. The gate 14 of the valve structure 17 being elevated in the diverting position, the obstruction of the passageway has been lifted. Moved to the diverting position, the valve structure 17 is also adapted to shut off the fluid connection between the inlet 13a and the sample outlet 13c. The transition segment 14d allows a smooth flow of the subsequent volume around the thinned portion of the stem and, at the same time, ensures that the thicker, lower portion of the stem 15 provides a plug to the sample outlet 13c. That is to say, the outer surface of lower portion of the stem 15 and the bore of the sample outlet 13c are fitted in size and shape to lie flush in the diverting position, thereby closing the sample outlet 13c. The skilled person is aware that a small clearance is needed and permissible to move the valve structure 17 back into the sampling position after the liquid flow has ceased and the sampled initial volume securely removed.

The outlet conduit 11 may be adapted for connection to a further receptacle for capturing the subsequent volume, or may be adapted for connection to a further liquid sampling device, e.g. a second sampling device in accordance with embodiments described hereinabove (and suitable outlet to inlet connectors) for sampling a fraction of the subsequent volume of the liquid flow, e.g. a fraction of the mid-stream urine.

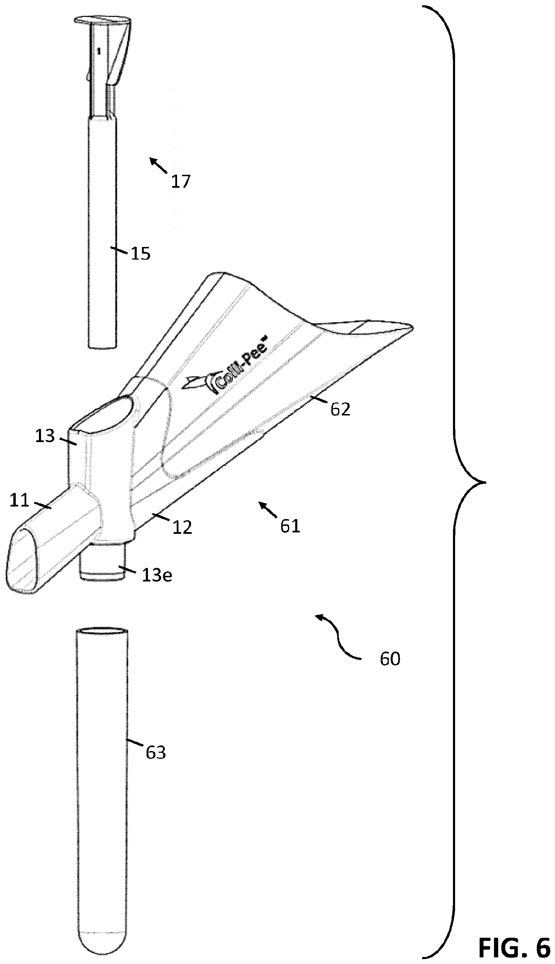

Components of the device 10 for sampling an initial volume of a liquid flow as described hereinabove, as well as attachable accessories, may be provided separately in a kit of parts. With reference to FIG. 6, an exploded view of such a kit 60 is shown. It is an advantage of the kit 60 that the parts can be manufactured separately before they are delivered to the user as a complete kit in a compact box (e.g. smaller than e.g. 380 mm x 265 mm x 32 mm, and shipped by regular mail), or as replacement parts for a previously purchased kit, who is assembling the sampling device according to instructions. The kit of parts 60 comprises the valve structure 17, the inlet conduit 12, the outlet conduit 11, the valve casing 13, and an optional receptacle 63. Preferably, the valve casing 13 and the inlet and outlet conduits 12, 11 are provided as a single integrally formed unit 61 such that both conduits 11, 12 are connected to the valve casing 13 and their proximate openings are permanently aligned with the inlet 13a and outlet 13b of the valve casing 13. A receptacle 63 (preferably with closing lid) is connectable to the valve casing 13 at a sample connector 13e thereof and has a collection volume equal to or larger than the initial volume of the liquid flow to be sampled, e.g. less than the first 15 ml of first-void urine, e.g. less than or equal to 4 ml of first-void urine. A dissolvable material layer of preservation agents or a buffer solution may be applied to a bottom of the receptacle 63 prior to the sampling of the initial volume. This allows preserving the sampled initial volume during the time required for sending the closed receptacle 63 to the laboratory charged with the urinalysis. Suitable materials for the components of the sampling device comprise, without being limited thereto, polypropylene, biodegradable polymers, or form-shaped bagasse, and withstand urine at body temperatures, e.g. at least 40 degree Celsius. Plastic components of the sampling device may be manufactured at large scale by injection molding. Preferably, the materials for the components of the sampling device are also easily compressed or folded such that they can be sent, as a kit or as separate parts, to the user by mail (e.g. when less than 28 mm thick), who then expands or unfolds the components for assembly. In embodiments of the invention, in which the inlet conduit 12 is not formed as a funnel 62, such an inlet conduit may be adapted to receive and secure a separate funnel at the distal opening, e.g. a funnel support may comprise a V-groove for placing and aligning a funnel and a clip for securing and maintaining an open configuration of the funnel. An accessory funnel may be part of the kit 60 and deliverable in compact form, e.g. folded or collapsed before being unfolded or expanded prior to use. A separate funnel has the advantage that it may be provided as a disposable funnel made from biodegradable polymers and suitable for direct flushing, in addition of being fitted to the anatomy of a specific gender, e.g. male or female. It is noticed that the valve structure 17 as one of the components of the kit 60 is removably insertable into the valve casing 13 at an opening at its top. In this case, the previously mentioned resilient stopper 13d is flexed under the pressure of the cap 16 when the valve structure 17 is being inserted. Once the cap 16 has passed the stopper 13d it snaps back to its original position, thus is restricting the axial movement of the inserted valve structure 17 to the interior of the valve casing 13 and prevents it from falling out. To facilitate insertion during assembly, the stopper 13d may have a downwards sloping upper side.

A variation of the preceding embodiment is now described with reference to FIG. 7. The kit of parts 70 comprises the valve structure 17, the inlet conduit 12, the outlet conduit 11, a bonnet 18, the valve casing 13, and an optional receptacle 73. Preferably, the bonnet 18 and the inlet and outlet conduits 12, 11 are provided as a single integrally formed unit 71 such that both conduits 11, 12 are connected to the bonnet 18 and have their proximate openings formed through the bonnet sidewalls. The present embodiment is different in that the valve casing 13 now takes the form of a sleeve that is inserted into and covered by the bonnet 18. It is understood from FIG. 6 that the valve structure 17 is first inserted into the valve casing 13 before the valve casing with valve structure is inserted into the bonnet 18, or the bonnet 18 is slipped thereover. The hollow interior of the valve casing 13 for the assembled sampling device is thus delimited by the base 13f of the valve casing 13, the double-walled section comprising the projecting side wall of the valve casing and the bonnet side wall, and the top wall of the bonnet. In consequence, the liquid passageway between inlet and outlet 13a, 13b, is completely enclosed, providing additional protection against spillage also on the top of the valve casing. The bonnet 18 may have a flexible annulus provided at its lower end portion, which annulus snap-fits the basis 13f of the valve casing 13 to securely fasten these two parts together. To facilitate sliding the bonnet 18 over the valve casing 13 and to ensure correct orientation, the bonnet 18 may be provided with a similar cylindrical shape as the valve casing 13, e.g. a generalized cylinder shape with asymmetrical base, e.g. a circular segment. This also allows the inlet 13a to be permanently aligned with the proximate opening of the inlet conduit 12 (formed through the bonnet) and the outlet 13b to be permanently aligned with the proximate opening of the outlet conduit 11 (also formed through the bonnet). A receptacle 73 (preferably with closing lid) is connectable to the valve casing 13 at a sample connector 13e thereof and has a collection volume equal to or larger than the initial volume of the liquid flow to be sampled, e.g. less than the first 15 ml of first-void urine, e.g. less than or equal to 4 ml of first-void urine. The sampling connector 13e which is shown in FIG. 7 has a baffle-forming recess provided at its lower end portion. As mention before, a dissolvable material layer of preservation agents or a buffer solution may be applied to a bottom of the receptacle 73 prior to the sampling of the initial volume.

The following describes a best mode of operating the device for sampling an initial volume of a liquid flow according to embodiments of the invention. To detect sexually transmitted infections in urine samples, samples taken from of a first small fraction of first-void urine, e.g. the initial 4 ml thereof, are recommended; this improves the reliability of the results obtained by urinalysis. Therefore, care has to be taken to not spill or lose any of the initial volume during the process of taking samples. This should not come at the expense of less handling comfort and hygiene when using the sampling device. Prior to sampling the initial volume of the liquid, e.g. urine, the user of the sampling device is connecting the receptacle to the valve casing (e.g. by screwing, snap-fitting, push-fitting the receptacle onto a sampling connector), or, if the receptacle has been previously connected to the valve casing, may check that it is connected in a secure way, e.g. such that it cannot loosen and fall down subsequently. In cases, in which the sampling device is delivered to the user as a kit of parts, optionally including further parts such as expandable funnels, the user is prompted to assemble these parts as described hereinabove. A buffer solution or perseveration agent may be added to the bottom of the receptacle. The user then positions the distant opening 12a of the inlet conduit 12 on the urinary meatus, wherein the wide funnel-shaped opening 12a is adapted to the anatomical shape around the meatus to prevent spillage or losses during the time urine is being execrated by the user's body and received by the inlet conduit 12. At the same time, the user is positioning the distant opening 11a of the outlet conduit 11 over or into a further recipient, e.g. into a larger container or a toilet. Next the user verifies that the sampling device is oriented nearly vertically and starts the urination process. An initial volume of first-void urine to the sampling device, where it is received by the inlet 13a and directed through the sampling outlet 13c into the receptacle. While receiving the initial volume, the lifting member 15a forces the valve structure 17 upwards until the cap 16 is pushing against the stopper 13d, e.g. until the valve structure has been moved from the sampling position to the diverting position. At that moment in time, the initial volume of urine has been sampled and the sample outlet 13c is being obstructed by the lower portion of the stem 15. However, the thinner portion of the stem permits the subsequent volume of urine to flow through the passageway in the valve casing 13 towards the outlet 13b and the distant opening of the outlet conduit 11a, where it is expelled from the sampling device. Once the urination is completed and no more urine is entering the inlet conduit 12, the user loosens the receptacle with the sampled initial volume. A closing lid can be firmly screwed or snapped onto the receptacle for safe delivery of the urine sample to the laboratory charged with the urinalysis and the detection of pathogens. The user may further dispose of the sampling device, e.g. by flushing a biodegradable device in the toilet, or may wash or rinse and disinfect the sampling device for a next use (optionally replacing accessories such as funnels).

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments. Other variations to the disclosed embodiments can be understood and effected by those skilled in the art in practicing the claimed invention, from a study of the drawings, the disclosure and the appended claims, which define the scope of the invention.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A device (10) for sampling an initial volume of a liquid flow, comprising:
- an inlet conduit (12) for receiving the liquid flow,
- an outlet conduit (11) for draining a subsequent volume of the liquid flow,
- a valve casing (13) arranged between the inlet conduit (12) and the outlet conduit (11) and having a passageway formed therethrough to fluidly connect the inlet conduit (12) to the outlet conduit (11), a sample outlet (13c) for draining the initial volume of the liquid flow extending through the valve casing (13) and into said passageway,
- a valve structure (17) arranged for axial movement in the valve casing (13) traverse to a direction of liquid flow through the passageway, the valve structure (17) comprising a gate (14) for obstructing the passageway and preventing the initial volume of the liquid flow from being transferred to the outlet conduit (11) when the valve structure (17) is in a sampling position, and the valve structure (17) further comprising a stem (15) connected to the gate (14) and adapted for obstructing the sample outlet (13c) without obstructing the passageway when the valve structure (17) is in a diverting position,
- a buoyancy-driven lifting member (15a) connected to the stem (15) for moving the valve structure (17) from the sampling position to the diverting position while the initial volume of the liquid flow is being sampled through the sample outlet (13c),
**characterized in that** at least one elongated groove (14a) is formed in a surface of the gate (14) facing the inlet conduit (12) and the at least one groove (14a) extends from the gate (14) to the stem (15), said at least one groove (14a) being adapted for receiving the initial volume of the liquid flow and for guiding said initial volume along the gate (14), the stem (15), and through the sample outlet (13c) towards a receptacle, connectable to the valve casing (13), when the valve structure (17) is in the sampling position.

2. The device (10) according to claim 1, wherein the buoyancy-driven lifting member (15a) comprises an air cavity formed in a lower portion of the stem (15).

3. The device (10) according to any of the previous claims, wherein the gate (14) comprises a slanted wall (14b) with edges lying flush with inner surfaces of the valve casing (13) when the valve structure (17) is in the sampling position.

4. The device (10) according to any of the previous claims, wherein the stem (15) is comprising a thinned upper portion, a wider lower portion, and a transition segment (14d) connected between the upper portion and the lower portion for tapering the thinner upper portion to the wider lower portion, the at least one groove (14a) extending into the upper portion of the stem (15) and an outer surface of the lower portion of the stem (15) lying flush with a bore of the sample outlet (13c) when the valve structure (17) is in the diverting position, ribbed edges optionally protruding from an outer surface of the upper portion of the stem (15), the ribbed edges being in a telescoping relationship with the bore of the sample outlet.

5. The device (10) according to any of the previous claims, wherein the valve casing (13) further comprises a sampling connector (13e) connected to a base (13f) of said valve casing (13) and at least partially circumferentially enclosing the sample outlet (13c) formed through said base (13f), the sampling connector (13e) optionally comprising an angled or baffle-forming opening at a lower end portion thereof, opposite to the base (13f).

6. The device (10) according to any of the previous claims, wherein the valve structure (17) further comprises a cap (16) connected to the gate (14), a rim of the cap (16) lying flush and being in a telescoping relationship with an inner surface of the valve casing (13), the rim of the cap (16) and the inner surface of the valve casing (13) optionally having a mating asymmetrical shape for preventing a rotational movement of the valve structure relative (17) to the valve casing (13).

7. The device (10) according to any of the previous claims, wherein the valve casing (13) comprises a resilient stopper (13d) protruding from an inner surface of said valve casing (13) and positioned to cooperate with an upper edge of the valve structure (17) such that a farther axial movement of the valve structure (17) is limited when the upper edge of the valve structure (17) is pushing against the stopper (13d) in the diverting position, and/or wherein the at least one groove (14a) is separated into two grooves by a stabilizing wall, and/or wherein the inlet conduit (12), outlet conduit (11), and the valve casing (13) are monolithically formed as a single part, proximate openings of the inlet conduit (12) and the outlet conduit (11) being respectively aligned with an inlet (13a) and an outlet (13b) of the passageway through the valve casing (13).

8. The device (10) according to any of the previous claims, further comprising a bonnet (18) connected between the inlet conduit (12) and the outlet conduit (11) and forming a lid to the valve casing (13), the bonnet (18) having proximate openings of the inlet conduit and outlet conduit formed therethrough, and the bonnet (18) being secured to a base (13f) of the valve casing (13) such that proximate openings of the inlet conduit (12) and the outlet conduit (11) are respectively aligned with an inlet (13a) and an outlet (13b) of the passageway through the valve casing (13).

9. A kit of parts (70) for sampling an initial volume of a liquid flow, comprising:
- a bonnet (18) having at least three openings,
- an inlet conduit (12) connectable to a first opening of the bonnet (18),
- an outlet conduit (11) connectable to a second opening of the bonnet (18),
- a valve casing (13) removably insertable into the bonnet (18) via a third opening therein, a base (13f) of the valve casing (13) being connectable to the third opening, the valve casing (13) having an inlet (13a) and an outlet (13b) formed therethrough defining a liquid passageway through the valve casing (13), a sample outlet (13c) extending through the base (13f) of the valve casing (13) and into the passageway, the valve casing (13) having an opening provided at a top side thereof, opposite to the base (13f),
- a valve structure (17) removably insertable into the valve casing (13), the valve structure (17) being arranged for axial movement in the valve casing (13), traverse to a direction of liquid flow through the passageway, the valve structure (17) comprising a gate (14) for obstructing the passageway when the valve structure (17) is in a sampling position, and the valve structure (17) further comprising a stem (15) connected to the gate (14) and adapted for obstructing the sample outlet without obstructing the passageway when the valve structure (17) is in a diverting position,
- a buoyancy-driven lifting member (15a) connectable to the stem (15);
**characterized by** at least one elongated groove (14a) being formed in a surface of the gate (14) and the at least one groove (14a) extending from the gate (14) into the stem (15), said at least one groove (14a) being adapted for, when the kit of parts is assembled and in use, receiving an initial volume of a liquid flow and for guiding said initial volume along the gate (14), the stem (15), and through the sample outlet (13c) towards a receptacle, connectable to the valve casing (13), when the valve structure (17) is in a sampling position.

10. A kit (70) according to claim 9, wherein the inlet conduit (12), outlet conduit (11), and the bonnet (18) are provided as a monolithic part.

11. A kit of parts (60) for sampling an initial volume of a liquid flow, comprising:
- a valve casing (13) having an inlet (13a) and an outlet (13b) formed therethrough defining a liquid passageway through the valve casing (13), a sample outlet (13c) extending through a base (13f) of the valve casing (13) and into the passageway, the valve casing (13) having an opening provided at a top side thereof, opposite to the base (13f),
- an inlet conduit (12) connectable or connected to the inlet of the valve casing (13),
- an outlet conduit (11) connectable or connected to the outlet of the valve casing (13), the inlet conduit (12), outlet conduit (11), and the valve casing (13) optionally being provided as a monolithic part,
- a valve structure (17) removably insertable into the valve casing (13), the valve structure (17) being arranged for axial movement in the valve casing (13), traverse to a direction of liquid flow through the passageway, the valve structure (17) comprising a gate (14) for obstructing the passageway when the valve structure (17) is in a sampling position, and the valve structure (17) further comprising a stem (15) connected to the gate (14) and adapted for obstructing the sample outlet (13c) without obstructing the passageway when the valve structure (17) is in a diverting position,
- a buoyancy-driven lifting member (15a) connectable to the stem (15);
**characterized by** at least one elongated groove (14a) being formed in a surface of the gate (14) and the at least one groove (14a) extending from the gate (14) into the stem (15), said at least one groove (14a) being adapted for, when the kit of parts is assembled and in use, receiving an initial volume of a liquid flow and for guiding said initial volume along the gate (14), the stem (15), and through the sample outlet (13c) towards a receptacle, connectable to the valve casing (13), when the valve structure (17) is in a sampling position.

12. A kit (60, 70) according to any of the claims 9 to 11, wherein the buoyancy-driven lifting member (15a) and the stem (15) of the valve structure (17) are provided as a monolithic part and the buoyancy-driven lifting member (15a) comprises an air cavity.

13. A kit (60, 70) according to any of the claims 9 to 12, wherein the gate (14) comprises a slanted wall with edges fitted to inner surfaces of the valve casing (13), and/or wherein the valve structure (17) further comprises a cap (16) connected to the gate (14), a rim of the cap (16) being fitted to an inner surface of the valve casing (13) to establish a telescoping relationship therewith, and/or wherein the valve casing (13) comprises a resilient stopper (13d) protruding from an inner surface of said valve casing (13) and positioned to cooperate with an upper edge of the valve structure (17) such that a farther axial movement of the valve structure (17) is limited when the upper edge of the valve structure (17) is pushing against the stopper (13d) in the diverting position.

14. A kit (60, 70) according to any of the claims 9 to 13, further comprising a receptacle (63, 73) with a volume ranging between 1 ml and 50 ml, the receptacle (63, 73) being connectable to a sampling connector (13e) provided at a lower side of the valve casing base (13f), and/or further comprising a funnel (62) connectable to the inlet conduit (12), the inlet conduit (12) being adapted for receiving and securing the funnel (62).

15. A kit (60, 70) according to any of the claims 9 to 14, wherein the inlet conduit (12) has a flexible shape, and/or wherein one or more parts of the kit (60, 70) are made from biodegradable polymers.

## Patentansprüche

1. Vorrichtung (10) zur Probenahme eines Anfangsvolumens eines Flüssigkeitsstroms, umfassend:
- eine Einlassleitung (12) zum Aufnehmen des Flüssigkeitsstroms,
- eine Auslassleitung (11) zum Ablassen eines nachfolgenden Volumens des Flüssigkeitsstroms,
- ein Ventilgehäuse (13), das zwischen der Einlassleitung (12) und der Auslassleitung (11) angeordnet ist und einen dort hindurch ausgebildeten Durchgang aufweist, um die Einlassleitung (12) mit der Auslassleitung (11) strömungstechnisch zu verbinden, wobei sich ein Probenauslass (13c) zum Ablassen des Anfangsvolumens des Flüssigkeitsstroms durch das Ventilgehäuse (13) und in den Durchgang erstreckt,
- eine Ventilstruktur (17), die für eine axiale Bewegung in dem Ventilgehäuse (13) quer zu einer Richtung des Flüssigkeitsstroms durch den Durchgang angeordnet ist, wobei die Ventilstruktur (17) ein Schieber (14) umfasst, um den Durchgang zu versperren und zu verhindern, dass das Anfangsvolumen des Flüssigkeitsstroms zu der Auslassleitung (11) weitergeleitet wird, wenn sich die Ventilstruktur (17) in einer Probenahmeposition befindet, und die Ventilstruktur (17) ferner einen Schaft (15) umfasst, der mit dem Schieber (14) verbunden ist und dazu eingerichtet ist, den Probenauslass (13c) zu versperren, ohne den Durchgang zu versperren, wenn sich die Ventilstruktur (17) in einer Umlenkposition befindet,
- ein auftriebsgetriebenes Hebeelement (15a), das mit dem Schaft (15) verbunden ist, um die Ventilstruktur (17) von der Probenahmeposition in die Umlenkposition zu bewegen, während eine Probe des Anfangsvolumens des Flüssigkeitsstroms durch den Probenauslass (13c) entnommen wird,
**dadurch gekennzeichnet, dass** mindestens eine längliche Nut (14a) in einer der Einlassleitung (12) zugewandten Oberfläche des Schiebers (14) ausgebildet ist und sich die mindestens eine Nut (14a) von dem Schieber (14) zu dem Schaft (15) erstreckt, wobei die mindestens eine Nut (14a) zum Aufnehmen des Anfangsvolumens des Flüssigkeitsstroms und zum Führen des Anfangsvolumens entlang des Schiebers (14), des Schafts (15) und durch den Probenauslass (13c) zu einem Behälter, der mit dem Ventilgehäuse (13) verbunden werden kann, eingerichtet ist, wenn sich die Ventilstruktur (17) in der Probenahmeposition befindet.

2. Vorrichtung (10) nach Anspruch 1, wobei das auftriebsgetriebene Hebeelement (15a) einen in einem unteren Abschnitt des Schafts (15) ausgebildeten Lufthohlraum umfasst.

3. Vorrichtung (10) nach einem der vorhergehenden Ansprüche, wobei der Schieber (14) eine abgeschrägte Wand (14b) aufweist, deren Kanten mit den Innenflächen des Ventilgehäuses (13) bündig sind, wenn sich die Ventilstruktur (17) in der Probenahmeposition befindet.

4. Vorrichtung (10) nach einem der vorhergehenden Ansprüche, wobei der Schaft (15) einen dünneren oberen Abschnitt, einen breiteren unteren Abschnitt und ein Übergangssegment (14d) umfasst, das zwischen dem oberen Abschnitt und dem unteren Abschnitt verbunden ist, um den dünneren oberen Abschnitt auf den breiteren unteren Abschnitt abzuschrägen, wobei sich die mindestens eine Nut (14a) in den oberen Abschnitt des Schafts (15) erstreckt und eine Außenfläche des unteren Abschnitts des Schafts (15) mit einer Bohrung des Probenauslasses (13c) bündig ist, wenn sich die Ventilstruktur (17) in der Umlenkposition befindet, wobei gerippte Kanten optional von einer Außenfläche des oberen Abschnitts des Schafts (15) vorstehen, wobei die gerippten Kanten in einer teleskopischen Beziehung mit der Bohrung des Probenauslasses stehen.

5. Vorrichtung (10) nach einem der vorhergehenden Ansprüche, wobei das Ventilgehäuse (13) ferner einen Probenahmeverbinder (13e) umfasst, der mit einer Basis (13f) des Ventilgehäuses (13) verbunden ist und den durch die Basis (13f) hindurch ausgebildeten Probenauslass (13c) zumindest teilweise in Umfangsrichtung umschließt, wobei der Probenahmeverbinder (13e) an einem unteren Endabschnitt desselben, der der Basis (13f) gegenüberliegt, optional eine abgewinkelte oder eine Prallplatte bildende Öffnung umfasst.

6. Vorrichtung (10) nach einem der vorhergehenden Ansprüche, wobei die Ventilstruktur (17) ferner eine Kappe (16) umfasst, die mit dem Schieber (14) verbunden ist, wobei ein Rand der Kappe (16) mit einer Innenfläche des Ventilgehäuses (13) bündig ist und in einer teleskopischen Beziehung zu dieser steht, wobei der Rand der Kappe (16) und die Innenfläche des Ventilgehäuses (13) optional eine zusammenpassende asymmetrische Form aufweisen, um eine Drehbewegung der Ventilstruktur (17) relativ zum Ventilgehäuse (13) zu verhindern.

7. Vorrichtung (10) nach einem der vorhergehenden Ansprüche, wobei das Ventilgehäuse (13) einen elastischen Stopper (13d) umfasst, der von einer Innenfläche des Ventilgehäuses (13) vorsteht und so positioniert ist, dass er mit einer oberen Kante der Ventilstruktur (17) so zusammenwirkt, dass eine weitere axiale Bewegung der Ventilstruktur (17) begrenzt ist, wenn die obere Kante der Ventilstruktur (17) in der Umlenkposition gegen den Stopper (13d) drückt, und/oder wobei die mindestens eine Nut (14a) durch eine stabilisierende Wand in zwei Nuten unterteilt ist, und/oder wobei die Einlassleitung (12), die Auslassleitung (11) und das Ventilgehäuse (13) monolithisch als ein einziges Teil ausgebildet sind, wobei nahegelegene Öffnungen der Einlassleitung (12) und der Auslassleitung (11) jeweils auf einem Einlass (13a) und einem Auslass (13b) des Durchgangs durch das Ventilgehäuse (13) ausgerichtet sind.

8. Vorrichtung (10) nach einem der vorhergehenden Ansprüche, ferner umfassend eine Haube (18), die zwischen der Einlassleitung (12) und der Auslassleitung (11) verbunden ist und einen Deckel für das Ventilgehäuse (13) bildet, wobei die Haube (18) naheliegende Öffnungen der Einlassleitung und der Auslassleitung aufweist, die durch sie hindurchgehen, und die Haube (18) an einer Basis (13f) des Ventilgehäuses (13) so befestigt ist, dass die nahegelegenen Öffnungen der Einlassleitung (12) und der Auslassleitung (11) jeweils auf einen Einlass (13a) und einen Auslass (13b) des Durchgangs durch das Ventilgehäuse (13) ausgerichtet sind.

9. Bausatz von Teilen (70) zur Probenahme eines Anfangsvolumens eines Flüssigkeitsstroms, umfassend:
- eine Haube (18) mit mindestens drei Öffnungen,
- eine Einlassleitung (12), die mit einer ersten Öffnung der Haube (18) verbunden werden kann,
- eine Auslassleitung (11), die mit einer zweiten Öffnung der Haube (18) verbunden werden kann,
- ein Ventilgehäuse (13), das in die Haube (18) über eine darin befindliche dritte Öffnung herausnehmbar eingesetzt werden kann, wobei eine Basis (13f) des Ventilgehäuses (13) mit der dritten Öffnung verbunden werden kann, wobei das Ventilgehäuse (13) einen jeweils dadurch hindurch ausgebildeten Einlass (13a) und einen Auslass (13b) aufweist, die einen Flüssigkeitsdurchgang durch das Ventilgehäuse (13) definieren, wobei sich ein Probenauslass (13c) durch die Basis (13f) des Ventilgehäuses (13) und in den Durchgang erstreckt, wobei das Ventilgehäuse (13) eine Öffnung aufweist, die an einer Oberseite desselben gegenüber der Basis (13f) vorgesehen ist,
- eine Ventilstruktur (17), die in das Ventilgehäuse (13) herausnehmbar eingesetzt werden kann, wobei die Ventilstruktur (17) für eine axiale Bewegung in dem Ventilgehäuse (13) quer zu einer Richtung des Flüssigkeitsstroms durch den Durchgang angeordnet ist, wobei die Ventilstruktur (17) einen Schieber (14) zum Versperren des Durchgangs, wenn sich die Ventilstruktur (17) in einer Probenahmeposition befindet, umfasst, und die Ventilstruktur (17) ferner einen Schaft (15) umfasst, der mit dem Schieber (14) verbunden ist und dazu eingerichtet ist, den Probenauslass zu versperren, ohne den Durchgang zu versperren, wenn sich die Ventilstruktur (17) in einer Umlenkposition befindet,
- ein auftriebsgetriebenes Hebeelement (15a), das mit dem Schaft (15) verbunden werden kann;
**gekennzeichnet durch** mindestens eine längliche Nut (14a), die in einer Oberfläche des Schieberteils (14) ausgebildet ist und die mindestens eine Nut (14a) sich von dem Schieber (14) in den Schaft (15) erstreckt, wobei, wenn der Bausatz von Teilen zusammengebaut und in Gebrauch ist, die mindestens eine Nut (14a) dazu eingerichtet ist, ein Anfangsvolumen eines Flüssigkeitsstroms aufzunehmen und das Anfangsvolumen entlang des Schiebers (14), des Schafts (15) und durch den Probenauslass (13c) zu einem Behälter zu führen, der mit dem Ventilgehäuse (13) verbunden werden kann, wenn sich die Ventilstruktur (17) in einer Probenahmeposition befindet.

10. Bausatz (70) nach Anspruch 9, wobei die Einlassleitung (12), die Auslassleitung (11) und die Haube (18) als ein monolithisches Teil vorgesehen sind.

11. Bausatz (60) zur Probenahme eines Anfangsvolumens eines Flüssigkeitsstroms, umfassend:
- ein Ventilgehäuse (13) mit einem durch dieses hindurch ausgebildeten Einlass (13a) und einem Auslass (13b), die einen Flüssigkeitsdurchgang durch das Ventilgehäuse (13) definieren, wobei sich ein Probenauslass (13c) durch eine Basis (13f) des Ventilgehäuses (13) und in den Durchgang hinein erstreckt, wobei das Ventilgehäuse (13) eine Öffnung aufweist, die an einer Oberseite desselben gegenüber der Basis (13f) vorgesehen ist,
- eine Einlassleitung (12), die mit dem Einlass des Ventilgehäuses (13) verbindbar oder verbunden ist,
- eine Auslassleitung (11), die mit dem Auslass des Ventilgehäuses (13) verbindbar oder verbunden ist, wobei die Einlassleitung (12), die Auslassleitung (11) und das Ventilgehäuse (13) optional als ein monolithisches Teil vorgesehen sind,
- eine Ventilstruktur (17), die in das Ventilgehäuse (13) herausnehmbar eingesetzt werden kann, wobei die Ventilstruktur (17) für eine axiale Bewegung in dem Ventilgehäuse (13) quer zu einer Richtung des Flüssigkeitsstroms durch den Durchgang angeordnet ist, wobei die Ventilstruktur (17) einen Schieber (14) zum Versperren des Durchgangs, wenn sich die Ventilstruktur (17) in einer Probenahmeposition befindet, umfasst, und die Ventilstruktur (17) ferner einen Schaft (15) umfasst, der mit dem Schieber (14) verbunden ist und dazu eingerichtet ist, den Probenauslass (13c) zu versperren, ohne den Durchgang zu versperren, wenn sich die Ventilstruktur (17) in einer Umlenkposition befindet,
- ein auftriebsgetriebenes Hebeelement (15a), das mit dem Schaft (15) verbunden werden kann;
**gekennzeichnet durch** mindestens eine längliche Nut (14a), die in einer Oberfläche des Schiebers (14) ausgebildet ist und die mindestens eine Nut (14a) sich von dem Schieber (14) in den Schaft (15) erstreckt, wobei, wenn der Bausatz zusammengebaut und in Gebrauch ist, die mindestens eine Nut (14a) dazu eingerichtet ist, ein Anfangsvolumen eines Flüssigkeitsstroms aufzunehmen und das Anfangsvolumen entlang des Schiebers (14), des Schafts (15) und durch den Probenauslass (13c) zu einem Behälter zu führen, der mit dem Ventilgehäuse (13) verbunden werden kann, wenn sich die Ventilstruktur (17) in einer Probenahmeposition befindet.

12. Bausatz (60, 70) nach einem der Ansprüche 9 bis 11, wobei das auftriebsgetriebene Hebeelement (15a) und der Schaft (15) der Ventilstruktur (17) als ein monolithisches Teil vorgesehen sind und das auftriebsgetriebene Hebeelement (15a) einen Lufthohlraum aufweist.

13. Bausatz (60, 70) nach einem der Ansprüche 9 bis 12, wobei der Schieber (14) eine abgeschrägte Wand mit Kanten aufweist, die an Innenflächen des Ventilgehäuses (13) angebracht sind, und/oder wobei der Ventilstruktur (17) ferner eine Kappe (16) aufweist, die mit dem Schieber (14) verbunden ist, wobei ein Rand der Kappe (16) an einer Innenfläche des Ventilgehäuses (13) angebracht ist, um eine teleskopische Beziehung mit diesem herzustellen, und/oder wobei das Ventilgehäuse (13) einen elastischen Stopper (13d) umfasst, der von einer Innenfläche des Ventilgehäuses (13) vorsteht und so positioniert ist, dass er mit einer oberen Kante der Ventilstruktur (17) so zusammenwirkt, dass eine weitere axiale Bewegung der Ventilstruktur (17) begrenzt ist, wenn die obere Kante der Ventilstruktur (17) in der Umlenkposition gegen den Stopper (13d) drückt.

14. Bausatz (60, 70) nach einem der Ansprüche 9 bis 13, der ferner ein Gefäß (63, 73) mit einem Volumen zwischen 1 ml und 50 ml umfasst, wobei das Gefäß (63, 73) mit einem Probenahmeverbinder (13e) verbunden werden kann, der an einer Unterseite der Basis (13f) des Ventilgehäuses vorgesehen ist, und/oder der ferner einen Trichter (62) umfasst, der mit der Einlassleitung (12) verbunden werden kann, wobei die Einlassleitung (12) zum Aufnehmen und Befestigen des Trichters (62) eingerichtet ist.

15. Bausatz (60, 70) nach einem der Ansprüche 9 bis 14, wobei die Einlassleitung (12) eine flexible Form aufweist, und/oder wobei ein oder mehrere Teile des Bausatzes (60, 70) aus biologisch abbaubaren Polymeren hergestellt sind.

## Revendications

1. Dispositif (10) d'échantillonnage d'un volume initial d'un écoulement de liquide, comprenant :
- une conduite d'entrée (12) pour recevoir l'écoulement de liquide,
- une conduite de sortie (11) pour drainer un volume ultérieur de l'écoulement de liquide,
- un logement de soupape (13) agencé entre la conduite d'entrée (12) et la conduite de sortie (11) et ayant un passage formé à travers celui-ci pour relier fluidiquement la conduite d'entrée (12) à la conduite de sortie (11), une sortie d'échantillon (13c) pour drainer le volume initial de l'écoulement de liquide s'étendant à travers le logement de soupape (13) et dans ledit passage,
- une structure de soupape (17) agencée pour un mouvement axial dans le logement de soupape (13) transversal à une direction d'écoulement de liquide à travers le passage, la structure de soupape (17) comprenant un obturateur (14) pour obstruer le passage et empêcher le volume initial de l'écoulement de liquide d'être transféré vers la conduite de sortie (11) lorsque la structure de soupape (17) est dans une position d'échantillonnage, et la structure de soupape (17) comprenant en outre une tige (15) reliée à l'obturateur (14) et adaptée pour obstruer la sortie d'échantillon (13c) sans obstruer le passage lorsque la structure de soupape (17) est dans une position de déviation,
- un organe (15a) de levage entraîné par poussée hydrostatique relié à la tige (15) pour déplacer la structure de soupape (17) de la position d'échantillonnage à la position de déviation pendant que le volume initial de l'écoulement de liquide est échantillonné à travers la sortie d'échantillon (13c),
**caractérisé en ce qu'**au moins une rainure allongée (14a) est formée dans une surface de l'obturateur (14) faisant face à la conduite d'entrée (12) et l'au moins une rainure (14a) s'étend de l'obturateur (14) à la tige (15), ladite au moins une rainure (14a) étant adaptée pour recevoir le volume initial de l'écoulement de liquide et pour guider ledit volume initial le long de l'obturateur (14), de la tige (15) et à travers la sortie d'échantillon (13c) vers un récipient, pouvant être relié au logement de soupape (13), lorsque la structure de soupape (17) est dans la position d'échantillonnage.

2. Dispositif (10) selon la revendication 1, dans lequel l'organe (15a) de levage entraîné par poussée hydrostatique comprend une cavité d'air formée dans une partie inférieure de la tige (15).

3. Dispositif (10) selon l'une quelconque des revendications précédentes, dans lequel l'obturateur (14) comprend une paroi inclinée (14b) dont les bords affleurent les surfaces internes du logement de soupape (13) lorsque la structure de soupape (17) est dans la position d'échantillonnage.

4. Dispositif (10) selon l'une quelconque des revendications précédentes, dans lequel la tige (15) comprend une partie supérieure amincie, une partie inférieure plus large et un segment de transition (14d) relié entre la partie supérieure et la partie inférieure pour effiler la partie supérieure plus fine vers la partie inférieure plus large, l'au moins une rainure (14a) s'étendant dans la partie supérieure de la tige (15) et une surface externe de la partie inférieure de la tige (15) affleurant un alésage de la sortie d'échantillon (13c) lorsque la structure de soupape (17) est dans la position de déviation, des bords nervurés faisant facultativement saillie depuis une surface externe de la partie supérieure de la tige (15), les bords nervurés étant dans une relation télescopique avec l'alésage de la sortie d'échantillon.

5. Dispositif (10) selon l'une quelconque des revendications précédentes, dans lequel le logement de soupape (13) comprend en outre un connecteur d'échantillonnage (13e) relié à une base (13f) dudit logement de soupape (13) et entourant au moins partiellement circonférentiellement la sortie d'échantillon (13c) formée à travers ladite base (13f), le connecteur d'échantillonnage (13e) comprenant facultativement une ouverture inclinée ou formant déflecteur au niveau de sa partie d'extrémité inférieure, opposée à la base (13f).

6. Dispositif (10) selon l'une quelconque des revendications précédentes, dans lequel la structure de soupape (17) comprend en outre un capuchon (16) relié à l'obturateur (14), un rebord du capuchon (16) affleurant et étant en relation télescopique avec une surface interne du logement de soupape (13), le rebord du capuchon (16) et la surface interne du logement de soupape (13) ayant facultativement une forme asymétrique correspondante pour empêcher un mouvement de rotation de la structure de soupape (17) par rapport au logement de soupape (13).

7. Dispositif (10) selon l'une quelconque des revendications précédentes, dans lequel le logement de soupape (13) comprend une butée élastique (13d) faisant saillie d'une surface interne dudit logement de soupape (13) et positionnée pour coopérer avec un bord supérieur de la structure de soupape (17) de sorte qu'un mouvement axial plus important de la structure de soupape (17) est limité lorsque le bord supérieur de la structure de soupape (17) pousse contre la butée (13d) en position de déviation, et/ou dans lequel l'au moins une rainure (14a) est séparée en deux rainures par une paroi de stabilisation, et/ou dans lequel la conduite d'entrée (12), la conduite de sortie (11) et le logement de soupape (13) sont formés de manière monolithique en une seule pièce, les ouvertures proches de la conduite d'entrée (12) et de la conduite de sortie (11) étant respectivement alignées avec une entrée (13a) et une sortie (13b) du passage à travers le logement de soupape (13).

8. Dispositif (10) selon l'une quelconque des revendications précédentes, comprenant en outre un chapeau (18) relié entre la conduite d'entrée (12) et la conduite de sortie (11) et formant un couvercle sur le logement de soupape (13), le chapeau (18) ayant des ouvertures proches de la conduite d'entrée et de la conduite de sortie formées à travers celui-ci, et le chapeau (18) étant fixé à une base (13f) du logement de soupape (13) de sorte que les ouvertures proches de la conduite d'entrée (12) et de la conduite de sortie (11) sont respectivement alignées avec une entrée (13a) et une sortie (13b) du passage à travers le logement de soupape (13).

9. Kit de pièces (70) pour l'échantillonnage d'un volume initial d'un écoulement de liquide, comprenant :
- un chapeau (18) ayant au moins trois ouvertures,
- une conduite d'entrée (12) pouvant être reliée à une première ouverture du chapeau (18),
- une conduite de sortie (11) pouvant être reliée à une deuxième ouverture du chapeau (18),
- un logement de soupape (13) pouvant être inséré de manière amovible dans le chapeau (18) via une troisième ouverture en son sein, une base (13f) du logement de soupape (13) pouvant être reliée à la troisième ouverture, le logement de soupape (13) ayant une entrée (13a) et une sortie (13b) formées à travers celui-ci définissant un passage de liquide à travers le logement de soupape (13), une sortie d'échantillon (13c) s'étendant à travers la base (13f) du logement de soupape (13) et dans le passage, le logement de soupape (13) ayant une ouverture prévue sur son côté supérieur, opposé à la base (13f),
- une structure de soupape (17) pouvant être insérée de manière amovible dans le logement de soupape (13), la structure de soupape (17) étant agencée pour un mouvement axial dans le logement de soupape (13), transversal à une direction d'écoulement de liquide à travers le passage, la structure de soupape (17) comprenant un obturateur (14) pour obstruer le passage lorsque la structure de soupape (17) est dans une position d'échantillonnage, et la structure de soupape (17) comprenant en outre une tige (15) reliée à l'obturateur (14) et adaptée pour obstruer la sortie d'échantillon sans obstruer le passage lorsque la structure de soupape (17) est dans une position de déviation,
- un organe (15a) de levage entraîné par poussée hydrostatique pouvant être relié à la tige (15) ;
**caractérisé par** au moins une rainure allongée (14a) formée dans une surface de l'obturateur (14) et l'au moins une rainure (14a) s'étendant de l'obturateur (14) jusque dans la tige (15), ladite au moins une rainure (14a) étant adaptée pour, lorsque le kit de pièces est assemblé et en utilisation, recevoir un volume initial d'un écoulement de liquide et pour guider ledit volume initial le long de l'obturateur (14), de la tige (15) et à travers la sortie d'échantillon (13c) vers un récipient, pouvant être relié au logement de soupape (13), lorsque la structure de soupape (17) est dans la position d'échantillonnage.

10. Kit (70) selon la revendication 9, dans lequel la conduite d'entrée (12), la conduite de sortie (11) et le chapeau (18) sont fournis sous la forme d'une pièce monolithique.

11. Kit de pièces (60) pour l'échantillonnage d'un volume initial d'un écoulement de liquide, comprenant :
- un logement de soupape (13) ayant une entrée (13a) et une sortie (13b) formées à travers celui-ci définissant un passage de liquide à travers le logement de soupape (13), une sortie d'échantillon (13c) s'étendant à travers la base (13f) du logement de soupape (13) et dans le passage, le logement de soupape (13) ayant une ouverture prévue sur son côté supérieur, opposé à la base (13f),
- une conduite d'entrée (12) reliée ou pouvant être reliée à l'ouverture du logement de soupape (13),
- une conduite de sortie (11) reliée ou pouvant être reliée à la sortie du logement de soupape (13), la conduite d'entrée (12), la conduite de sortie (11) et le logement de soupape (13) étant facultativement fournis sous la forme d'une pièce monolithique,
- une structure de soupape (17) pouvant être insérée de manière amovible dans le logement de soupape (13), la structure de soupape (17) étant agencée pour un mouvement axial dans le logement de soupape (13), transversal à une direction d'écoulement de liquide à travers le passage, la structure de soupape (17) comprenant un obturateur (14) pour obstruer le passage lorsque la structure de soupape (17) est dans une position d'échantillonnage, et la structure de soupape (17) comprenant en outre une tige (15) reliée à l'obturateur (14) et adaptée pour obstruer la sortie d'échantillon (13c) sans obstruer le passage lorsque la structure de soupape (17) est dans une position de déviation,
- un organe (15a) de levage entraîné par poussée hydrostatique pouvant être relié à la tige (15) ;
**caractérisé par** au moins une rainure allongée (14a) formée dans une surface de l'obturateur (14) et l'au moins une rainure (14a) s'étendant de l'obturateur (14) jusque dans la tige (15), ladite au moins une rainure (14a) étant adaptée pour, lorsque le kit de pièces est assemblé et en utilisation, recevoir un volume initial d'un écoulement de liquide et pour guider ledit volume initial le long de l'obturateur (14), de la tige (15) et à travers la sortie d'échantillon (13c) vers un récipient, pouvant être relié au logement de soupape (13), lorsque la structure de soupape (17) est dans la position d'échantillonnage.

12. Kit (60, 70) selon l'une quelconque des revendications 9 à 11, dans lequel l'organe (15a) de levage entraîné par poussée hydrostatique et la tige (15a) de la structure de soupape (17) sont fournis sous la forme d'une pièce monolithique et l'organe (15a) de levage entraîné par poussée hydrostatique comprend une cavité d'air.

13. Kit (60, 70) selon l'une quelconque des revendications 9 à 12, dans lequel l'obturateur (14) comprend une paroi inclinée dont les bords s'ajustent aux surfaces internes du logement de soupape (13), et/ou la structure de soupape (17) comprend en outre un capuchon (16) relié à l'obturateur (14), un rebord du capuchon (16) s'ajustant avec une surface interne du logement de soupape (13) pour établir une relation télescopique avec celle-ci, et/ou le logement de soupape (13) comprend une butée élastique (13d) faisant saillie d'une surface interne dudit logement de soupape (13) et positionnée pour coopérer avec un bord supérieur de la structure de soupape (17) de sorte qu'un mouvement axial plus important de la structure de soupape (17) est limité lorsque le bord supérieur de la structure de soupape (17) pousse contre la butée (13d) en position de déviation.

14. Kit (60, 70) selon l'une quelconque des revendications 9 à 13, comprenant en outre un récipient (63, 73) d'un volume compris entre 1 ml et 50 ml, le récipient (63, 73) pouvant être relié à un connecteur d'échantillonnage (13e) prévu sur un côté inférieur de la base (13f) de logement de soupape, et/ou comprenant en outre un entonnoir (62) pouvant être relié à la conduite d'entrée (12), la conduite d'entrée (12) étant adaptée pour recevoir et fixer l'entonnoir (62).

15. Kit (60, 70) selon l'une quelconque des revendications 9 à 14, dans lequel la conduite d'entrée (12) a une forme flexible, et/ou une ou plusieurs pièces du kit (60, 70) sont fabriquées à partir de polymères biodégradables.
